# FASCICULE DE BREVET EUROPEEN

(11) **EP 3 302 232 B1**
(45) Date de publication et mention de la délivrance du brevet: **09.08.2023**
(21) Numéro de dépôt: 16727453.9
(22) Date de dépôt: 01.06.2016
(51) Int. Cl.: A61B 5/00, A61B 8/08

(54) **DISPOSITIF NON INVASIF DE DETECTION DE LESION HEPATIQUE**
NICHTINVASIVE VORRICHTUNG ZUR ERKENNUNG VON LEBERSCHÄDEN
NON-INVASIVE DEVICE FOR DETECTING LIVER DAMAGE

(30) Priorité: 02.06.2015 FR 1554995
(43) Date de publication de la demande: 11.04.2018
(60) Demande divisionnaire: 23172405.5
(73) Titulaire: Echosens, 75014 Paris (FR)
(72) Inventeur: MIETTE, Véronique, 94800 Villejuif (FR); SANDRIN, Laurent, 94340 Bourg-la-Reine (FR); SASSO, Magali, 30127 Bellegarde (FR)
(74) Mandataire: Cabinet Camus Lebkiri
(86) Numéro de dépôt international: PCT/EP2016/062392
(87) Numéro de publication internationale: WO 2016/193312

(56) Documents cités:
- WO-A1-2013/025798
- WO-A2-2015/014763
- MIETTE V ET AL: "P1C-2 Liver Stiffness Measurement Using Transient Elastography in Patients with Non-Alcoholic Fatty Liver (NAFLD) and Non-Alcoholic Steatohepatitis (NASH)", ULTRASONICS SYMPOSIUM, 2007. IEEE, IEEE, PISCATAWAY, NJ, USA, 1 octobre 2007 (2007-10-01), pages 1333-1336, XP031195226, DOI: 10.1109/ULTSYM.2007.335 ISBN: 978-1-4244-1383-6
- SASSO M ET AL: "Controlled Attenuation Parameter (CAP): A Novel VCTE(TM) Guided Ultrasonic Attenuation Measurement for the Evaluation of Hepatic Steatosis: Preliminary Study and Validation in a Cohort of Patients with Chronic Liver Disease from Various Causes", ULTRASOUND IN MEDICINE AND BIOLOGY, NEW YORK, NY, US, vol. 36, no. 11, 1 November 2010 (2010-11-01), pages 1825-1835, XP027430252, ISSN: 0301-5629, DOI: 10.1016/J.ULTRASMEDBIO.2010.07.005 [retrieved on 2010-09-26]
- PHILIP N NEWSOME ET AL: "FibroScan-AST (FAST) score for the non-invasive identification of patients with non-alcoholic steatohepatitis with significant activity and fibrosis: a prospective derivation and global validation study", THE LANCET - GASTROENTEROLOGY & HEPATOLOGY, vol. 5, no. 4, 11 March 2020 (2020-03-11), pages 362-373, XP055761789, US ISSN: 2468-1253, DOI: 10.1016/S2468-1253(19)30383-8

## Description

### DOMAINE TECHNIQUE

L'invention concerne un dispositif non invasif de détection de lésion hépatique utilisant les ondes ultrasonores et les ondes de cisaillement. Ce dispositif peut être utilisé pour l'homme et pour l'animal et est par exemple destiné à la détection d'une lésion hépatique de type stéato-hépatite d'origine alcoolique (également connue sous l'acronyme ASH pour Alcoholic SteatoHepatitis en anglais) ou non alcoolique (également connue sous l'acronyme NASH pour Non Alcoholic Steato Hepatitis en anglais). L'invention se rapporte également à un score reflétant une lésion hépatique.

### ART ANTERIEUR

Habituellement, les maladies chroniques du tissu hépatique génèrent des lésions hépatiques telles que la fibrose. La fibrose est un processus de cicatrisation fibreux du tissu hépatique résultant d'une inflammation. L'évolution de la fibrose initialement asymptomatique peut évoluer vers une cirrhose. L'élasticité du tissu hépatique constitue un marqueur de la fibrose hépatique. Afin de mesurer et quantifier l'élasticité du tissu hépatique, il est connu d'utiliser l'élastographie impulsionnelle, comme décrit, par exemple, dans la demande de brevet numéro FR 2843290.

Ce document présente une mise en oeuvre d'un dispositif selon l'art antérieur. Ce dispositif est composé d'une sonde munie d'un générateur de vibration générant une onde élastique basse fréquence dans un tissu, par exemple par vibration, et analysant la propagation de cette onde élastique basse fréquence à l'aide d'ondes ultrasonores haute fréquence émises et reçues par un transducteur ultrasonore. Les mesures obtenues via ce dispositif permettent de quantifier l'élasticité du tissu hépatique. Ce dispositif permet également de quantifier l'atténuation ultrasonore des tissus, comme décrit, par exemple, dans la demande de brevet numéro FR 2949965. La quantification de l'atténuation ultrasonore dans le foie est corrélée la quantité de stéatose.

Des exemples de l'art antérieur de méthodes d'évaluation de la fibrose et de la stéatose, en particulier chez des sujets atteints de maladies stéato-hépatiques telle que la NASH, peuvent être trouvés dans MIETTE ET AL. « Liver Stiffness measurement using transient elastography in patients with non-alcoholic fatty liver (NAFLD) and non-alcoholic steatohepatitis (NASH) », IEEE ULTRASONICS SYMPOSIUM, 2007 et dans WO 2013/025798 A1.

En revanche, chez l'homme certaines maladies, par exemple la NASH, ne sont pas nécessairement liées qu'à la seule quantité de fibrose ou à la seule quantité de stéatose, et peut par exemple associer des lésions de type stéatose (présence de graisse dans le foie) et inflammation avec ou sans fibrose. Par conséquent, le stade de NASH ou l'évolution vers la NASH ne peut pas être diagnostiqué au moyen d'un seul paramètre.

Des exemples de systèmes de l'art antérieur pour calculer un score humain ou animal, en particulier relatif à un état de santé du foie, peuvent être trouvés dans WO2015/014763 A2.

### EXPOSE DE L'INVENTION

La présente invention vise à résoudre au moins un des inconvénients de l'état de la technique précités. Pour cela, l'invention propose un dispositif non invasif de détection de lésion hépatique prenant en compte différents paramètres. L'invention propose également un score reflétant un type de lésion hépatique.

A cette fin, un aspect de l'invention a pour objet un dispositif de calcul d'un score pour l'homme ou l'animal ledit score étant une évaluation quantitative ou semi-quantitative d'une lésion hépatique de type stéato-hépatite d'origine alcoolique ou non-alcoolique selon la revendication 1.

Ce mode de réalisation présente notamment l'avantage de permettre une détection précoce de certains types de lésion hépatique, comme par exemple la NASH, la NASH pouvant être corrélée à une inflammation, à une fibrose et à une stéatose. En revanche, la NAFLD (pour Non-Alcoholic Fatty Liver Disease) est corrélée simplement à la stéatose. Grâce au score, il est donc possible de différencier des patients atteints d'une maladie de type NAFLD des patients atteints d'une maladie de type NASH.

Des modes de réalisation de l'invention concernent un dispositif selon l'une des revendications 2 à 9.

Dans une mise en oeuvre non limitative, le dispositif selon l'invention est construit et agencé pour délivrer le score de manière concomitante aux paramètres physiques mesurés. En d'autres termes, l'échographe ou le dispositif construit et agencé pour mesurer au moins l'élasticité hépatique mesure des paramètres physiques et le dispositif selon l'invention calcule le score en prenant compte au moins des paramètres physiques mesurés.

Dans une mise en oeuvre non limitative du dispositif de calcul selon l'invention, le dispositif de calcul est construit et agencé pour communiquer avec :
- un échographe distant, ou
- un dispositif distant construit et agencé pour mesurer au moins l'élasticité hépatique.

Dans une mise en oeuvre non limitative du dispositif selon l'invention, le paramètre corrélé à la stéatose est une mesure de l'atténuation des ondes ultrasonores, par exemple le paramètre appelé CAP tel que décrit dans l'article Sasso, M., et al. (2010). "Controlled atténuation parameter (CAP): a novel VCTE guided ultrasonic atténuation measurement for the évaluation of hepatic steatosis: preliminary study and validation in a cohort of patients with chronic liver disease from various causes." Ultrasound Med Biol 36(11): 1825-1835.

Dans une mise en oeuvre non limitative du dispositif selon l'invention, le paramètre corrélé à la stéatose est une mesure de viscosité du tissu hépatique.

Dans une mise en oeuvre non limitative du dispositif selon l'invention, le dispositif de calcul est construit et agencé pour communiquer avec un dispositif d'affichage du score. Le score peut être affiché sous la forme d'une valeur numérique, d'un indicateur binaire, une probabilité ou un risque. Ce mode de réalisation présente notamment l'avantage de permettre une simplicité d'interprétation de l'analyse du score reflétant un état d'une liaison hépatique calculée.

### BREVE DESCRIPTION DES FIGURES

D'autres caractéristiques et avantages de l'invention ressortiront clairement de la description qui en est donnée ci-après, à titre indicatif et nullement limitatif, en référence :
- à la figure 1 illustrant, de façon schématique, un premier exemple de réalisation d'un dispositif de calcul d'un score reflétant un état d'une lésion hépatique intégré dans un dispositif construit et agencé pour mesurer l'élasticité hépatique,
- à la figure 2 illustrant, de façon schématique, un deuxième exemple de réalisation d'un dispositif de calcul d'un score reflétant un état d'une lésion hépatique construit et agencé pour communiquer avec un échographe distant.

### DESCRIPTION DE L'INVENTION

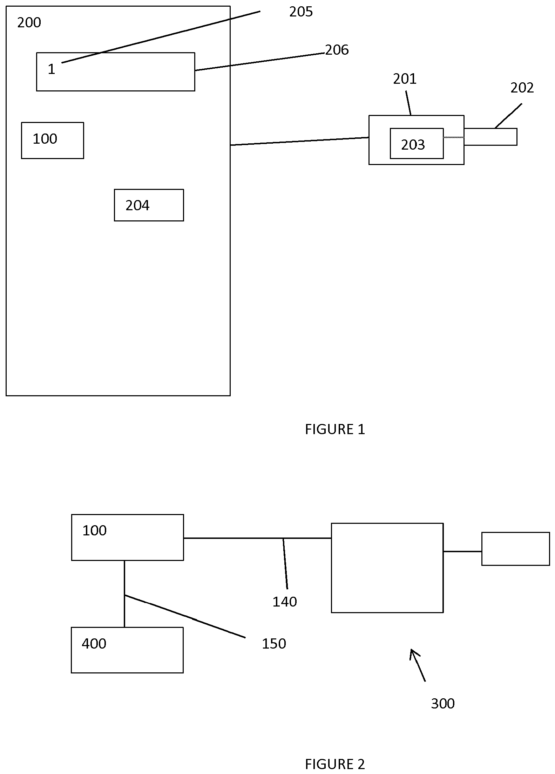

La figure 1 représente un dispositif 100 de calcul d'un score reflétant un état d'une lésion hépatique intégré dans un dispositif 200 construit et agencé pour mesurer l'élasticité hépatique.

Dans ce mode de réalisation non limitatif, le dispositif 200 comporte une sonde d'élastographie 201 munie d'un transducteur ultrasonore 202 construit et agencé pour émettre et recevoir des ondes ultrasonores. Dans ce mode de réalisation, la sonde d'élastographie 201 comporte en outre des moyens pour générer une onde de cisaillement dans le tissu hépatique. Lesdits moyens peuvent être un actionneur électrodynamique 203 construit et agencé pour générer une onde basse fréquence. Le dispositif 200 est donc construit et agencé pour mesurer des paramètres physiques, par exemple des paramètres qui sont corrélés à l'inflammation et/ou à la fibrose et des paramètres qui sont corrélés à la stéatose.

A titre d'exemple, un paramètre lié à la fibrose peut être l'élasticité du foie. Cette mesure d'élasticité constitue un marqueur de l'état pathologique du tissu hépatique.

Le paramètre corrélé à la stéatose peut être une mesure de l'atténuation des ondes ultrasonores dans le tissu hépatique. La stéatose hépatique est une accumulation de graisse dans le foie. La mesure de l'atténuation de la propagation des ondes ultrasonores permet donc de quantifier la stéatose.

Le dispositif 100 de calcul d'un score reflétant un état d'une lésion hépatique est construit et agencé pour calculer un score au moyen d'un paramètre corrélé à l'inflammation du tissu hépatique et/ou un paramètre corrélé à la fibrose. Dans l'exemple décrit, ces paramètres sont mesurés au moyen de la sonde d'élastographie 201.

Dans l'exemple illustré, le dispositif 200 comporte également une interface homme-machine 204 construite et agencée pour entrer des paramètres marqueurs du syndrome métabolique utilisés pour le calcul du score.

Ainsi, un opérateur peut entrer, via l'interface homme-machine 204, des paramètres marqueurs du syndrome métabolique. On entend par syndrome métabolique, l'association d'une série de problèmes de santé ayant en commun un mauvais métabolisme corporel, il s'agit d'un regroupement de facteurs de risque plus ou moins liés par une origine, des cibles métaboliques ou des mécanismes communs. Ce groupe de paramètres peut ainsi comporter : le cholestérol HDL, les triglycérides, la glycémie, la tension artérielle, et/ou le tour de taille.

Cette interface homme-machine 204 est également construite et agencée pour entrer des paramètres biologiques utilisés pour le calcul du score. Ces paramètres biologiques peuvent être : les transaminases (ALAT, ASAT), GGT, PAL, Fer sérique, cholestérol, cholestérol HDL, glycémie, insulinémie, bilirubine, a2macroglobuline, haptoglobine, apolipoprotéine A1, CK18, triglycérides, adiponectine, et/ou leptine.

Cette interface homme-machine 204 est également construite et agencée pour entrer des paramètres anthropomorphiques et démographiques utilisés pour le calcul du score. Ces paramètres démographiques et anthropomorphiques sont par exemple formés par l'âge, le sexe, la taille, le poids, le tour de taille, le tour de hanche ou le périmètre thoracique d'une personne.

En fonction de ces différents paramètres, le dispositif de calcul 100 calcule un score au moyen d'une régression logistique ou toute autre méthode de scoring, par exemple du type arbres de décisions, classificateur bayésien, forêts aléatoires, arbres de décision séparateurs à vaste marge (SVM), ou encore réseau de neurone.

A cette fin, le dispositif de calcul 100 peut être formé par un ou plusieurs microprocesseurs construit(s) et adapté(s) pour exécuter des séquences d'instructions permettant une mise en oeuvre de la régression logistiques précitée ou toute autre méthode de scoring.

Dans l'exemple illustré, le score calculé est représenté sous la forme d'un indicateur binaire 205 égal à 1 et affiché sur un écran 206 du dispositif 200. Cet indicateur binaire 205 peut être utilisé pour recommander à un patient de réaliser une consultation auprès d'un spécialiste. Par exemple, lorsque l'indicateur est égal à 1, le patient est diagnostiqué comme étant à risque et nécessite une investigation plus poussée ou des examens complémentaires doivent être réalisés.

A contrario, lorsque l'indicateur est égal à 0, le patient ne doit pas consulter de spécialiste. Cet indicateur peut également être différent, il peut être implémenté sous la forme d'une valeur.

Dans cette réalisation non limitative, les mesures de paramètres physiques, l'entrée d'autres paramètres, le calcul du score et l'affichage du score sont réalisés sur le dispositif 200. Ainsi, ce mode de réalisation présente notamment l'avantage de calculer en temps réel le score (autrement dit sur le lieu où sont réalisées les mesures des paramètres physiques), puis d'afficher le score permettant ainsi une rapidité d'analyse.

Dans des exemples différents non limitatifs, le dispositif 200 peut être formé par un échographe, un IRM, ou un IRM mettant en oeuvre l'élastographie par résonance magnétique (MRE).

Dans une mise en oeuvre non limitative illustrée sur la figure 2, le dispositif de calcul d'un score reflétant un état d'une lésion hépatique 100 est construit et agencé pour communiquer avec un échographe 300 distant. Autrement-dit, le dispositif de calcul 100 est déporté vis-à-vis de l'échographe 300. Ainsi, les mesures sont réalisés sur l'échographe 300 puis transmises par une liaison réseau 140, par exemple une liaison de type Ethernet ou Bluetooth ou Wi-Fi, au dispositif de calcul 100. Il est également possible de transmettre d'autres paramètres, par exemple de type anthropomorphiques ou démographiques, au dispositif de calcul 100 via un ordinateur 400. De façon similaire, cet ordinateur 400 peut communiquer avec le dispositif de calcul 100 via une liaison Ethernet ou Wi-Fi 150. Le dispositif de calcul 100 peut être matérialisé par un ou plusieurs processeurs. Par ailleurs, l'ordinateur peut être intégré dans l'échographe 300.

Dans cette réalisation non limitative, l'affichage du score peut être réalisé sur l'échographe 300, sur l'ordinateur 400 ou les deux.

## Revendications

1. Dispositif de calcul (100) d'un score (205) pour l'homme ou l'animal ledit score (205) étant une évaluation quantitative ou semi-quantitative d'une lésion hépatique de type stéato-hépatite d'origine alcoolique ou non-alcoolique, ledit dispositif de calcul (100) étant construit et agencé pour calculer un score (205) au moyen des paramètres au moins physiques voire biologiques suivants :
- Un paramètre corrélé à la fibrose,
- Un paramètre corrélé à la stéatose, et
- Un paramètre supplémentaire corrélé à l'activité inflammatoire,
le paramètre supplémentaire corrélé à l'activité inflammatoire étant choisi parmi : ALAT, ASAT, les GGT,
le paramètre corrélé à la fibrose étant l'élasticité,
le paramètre corrélé à la stéatose étant une mesure de l'atténuation des ondes ultrasonores ou de la viscosité hépatique,
le dispositif calculant le score en fonction desdits paramètres, le score étant calculé au moyen d'une régression logistique.

2. Dispositif de calcul (100) selon la revendication 1 **caractérisé en ce qu'**il est embarqué dans :
- un échographe, ou
- un dispositif construit et agencé pour mesurer au moins l'élasticité hépatique.

3. Dispositif de calcul (100) selon la revendication 2 précédente **caractérisé en ce qu'**il est construit et agencé pour délivrer le score de manière concomitante aux paramètres physiques mesurés

4. Dispositif de calcul (100) selon la revendication1 **caractérisé en ce qu'**il est construit et agencé pour communiquer avec :
- un échographe distant, ou
- un dispositif distant construit et agencé pour mesurer au moins l'élasticité hépatique.

5. Dispositif de calcul (100) selon l'une quelconque des revendications précédentes **caractérisé en ce qu'**il est construit et agencé pour calculer le score au moyen d'au moins un paramètre supplémentaire corrélé au syndrome métabolique.

6. Dispositif de calcul (100) selon l'une quelconque des revendications précédentes **caractérisé en ce qu'**il est construit et agencé pour calculer le score au moyen d'au moins un paramètre supplémentaire de type anthropomorphique.

7. Dispositif de calcul (100) selon l'une quelconque des revendications précédentes **caractérisé en ce qu'**il est construit et agencé pour calculer le score au moyen d'au moins un paramètre supplémentaire de type biologique.

8. Dispositif de calcul (100) selon l'une quelconque des revendications précédentes **caractérisé en ce qu'**il est construit et agencé pour communiquer avec un dispositif d'affichage (206) du score (205).

9. Dispositif de calcul (100) selon la revendication précédente **caractérisé en ce que** le score (205) est affiché sous la forme d'une valeur numérique, d'un indicateur binaire, une probabilité ou un risque.

## Patentansprüche

1. Berechnungsvorrichtung (100) eines Scores (205) für Menschen oder Tiere, wobei dieser Score (205) eine quantitative oder halb-quantitative Evaluierung einer Lebererkrankung vom Typ alkoholbedingte bzw. nicht alkoholbedingte Steatohepatitis ist, wobei die Berechnungsvorrichtung (100) mittels folgender zumindest physikalischer bzw. biologischer Parameter aufgebaut und angeordnet ist, um einen Score (205) zu berechnen:
- einen mit der Fibrose korrelierten Parameter,
- einen mit der Steatose korrelierten Parameter, und
- einen mit der Entzündungsaktivität korrelierten zusätzlichen Parameter, wobei der mit der Entzündungsaktivität korrelierte zusätzliche Parameter ausgewählt wird unter: ALAT, ASAT, den GGT,
wobei der mit der Fibrose korrelierte Parameter die Elastizität ist,
wobei der mit der Steatose korrelierte Parameter eine Messung der Schwächung der Ultraschallwellen oder der Viskosität der Leber ist,
wobei die Vorrichtung den Score in Abhängigkeit dieser Parameter berechnet und der Score mittels einer logistischen Regression berechnet wird.

2. Berechnungsvorrichtung (100) nach Anspruch 1, **dadurch gekennzeichnet, dass** sie eingebettet ist in:
- einem Ultraschallgerät, oder
- einer aufgebauten und angeordneten Vorrichtung, um mindestens die Elastizität der Leber zu messen.

3. Berechnungsvorrichtung (100) nach Anspruch 2, **dadurch gekennzeichnet, dass** sie aufgebaut und angeordnet ist, um begleitend zu den gemessenen physikalischen Parametern den Score zu liefern.

4. Berechnungsvorrichtung (100) nach Anspruch 1, **dadurch gekennzeichnet, dass** sie aufgebaut und angeordnet ist, um zu kommunizieren mit:
- einem entfernten Ultraschallgerät, oder
- einer entfernten Vorrichtung, die aufgebaut und angeordnet ist, um mindestens die Elastizität der Leber zu messen.

5. Berechnungsvorrichtung (100) nach einem der vorhergehenden Ansprüche, **dadurch gekennzeichnet, dass** sie aufgebaut und angeordnet ist, um den Score mittels mindestens eines mit dem metabolischen Syndrom korrelierten zusätzlichen Parameters zu berechnen.

6. Berechnungsvorrichtung (100) nach einem der vorhergehenden Ansprüche, **dadurch gekennzeichnet, dass** sie aufgebaut und angeordnet ist, um den Score mittels mindestens eines zusätzlichen Parameters anthropomorpher Art zu berechnen.

7. Berechnungsvorrichtung (100) nach einem der vorhergehenden Ansprüche, **dadurch gekennzeichnet, dass** sie aufgebaut und angeordnet ist, um den Score mittels mindestens eines zusätzlichen Parameters biologischer Art zu berechnen.

8. Berechnungsvorrichtung (100) nach einem der vorhergehenden Ansprüche, **dadurch gekennzeichnet, dass** sie aufgebaut und angeordnet ist, um mit einer Anzeigevorrichtung (206) des Scores (205) zu kommunizieren.

9. Berechnungsvorrichtung (100) nach vorhergehendem Anspruch, **dadurch gekennzeichnet, dass** der Score (205) in Form eines Zahlenwerts, eines binären Indikators, einer Wahrscheinlichkeit oder eines Risikos angezeigt wird.

## Claims

1. Device for calculating (100) a score (205) for a human or an animal, said score (205) being a quantitative or semi-quantitative evaluation of liver damage of alcoholic or non-alcoholic steatohepatitis type, said calculating device (100) being constructed and arranged to calculate a score (205) using the following at least physical or even biological parameters:
- a parameter correlated to fibrosis,
- a parameter correlated to steatosis, and
- an additional parameter correlated to inflammatory activity,
the additional parameter correlated to inflammatory activity being selected from: ALAT, ASAT, GGTs,
the parameter correlated to fibrosis being elasticity,
the parameter correlated to steatosis being a measurement of the attenuation of ultrasonic waves or of liver viscosity,
the calculating device calculating the score as a function of said parameters, the score being calculated based on a logistic regression.

2. Calculating device (100) according to claim 1 **characterised in that** it is integrated in:
- an ultrasound scanner, or
- a device constructed and arranged to measure at least liver elasticity.

3. Calculating device (100) according to claim 2 **characterised in that** it is constructed and arranged to deliver the score concurrently with the measured physical parameters.

4. Calculating device (100) according to claim 1 **characterised in that** it is constructed and arranged to communicate with:
- a remote ultrasound scanner, or
- a remote device constructed and arranged to measure at least liver elasticity.

5. Calculating device (100) according to any of the preceding claims **characterised in that** it is constructed and arranged to calculate the score using at least one additional parameter corresponding to metabolic syndrome.

6. Calculating device (100) according to any of the preceding claims **characterised in that** it is constructed and arranged to calculate the score using at least one additional parameter of anthropomorphic type.

7. Calculating device (100) according to any of the preceding claims **characterised in that** it is constructed and arranged to calculate the score using at least one additional parameter of biological type.

8. Calculating device (100) according to any of the preceding claims **characterised in that** it is constructed and arranged to communicate with a display device (206) for displaying the score (205).

9. Calculating device (100) according to the preceding claim **characterised in that** the score (205) is displayed in the form of a numerical value, a binary indicator, a probability or a risk.
